# EUROPÄISCHE PATENTSCHRIFT

(11) **EP 2 226 010 B1**
(45) Veröffentlichungstag und Bekanntmachung des Hinweises auf die Patenterteilung: **18.06.2014**
(21) Anmeldenummer: 10001200.4
(22) Anmeldetag: 05.02.2010
(51) Int. Cl.: A61B 6/04

(54) **Patientenlagerungstisch für die Radiologie**
Patient table for radiology
Table de traitement pour la radiologie

(30) Priorität: 04.03.2009 DE 202009002897 U
(43) Veröffentlichungstag der Anmeldung: 08.09.2010
(73) Patentinhaber: Roesys GmbH, 32339 Espelkamp (DE)
(72) Erfinder: Affeld, Dietrich, 32479 Hille (DE)
(74) Vertreter: Rolf, Gudrun

(56) Entgegenhaltungen:
- EP-A2- 0 923 922
- WO-A1-01/72226

## Beschreibung

Die Erfindung betrifft einen Patientenlagerungstisch für die Radiologie gemäß dem Oberbegriff des Hauptanspruches.

Es ist ein solcher Patientenlagerungstisch für Aufnahmeplätze bekannt, DE 196 36 906 A1, der zwei Hubsäulen mit Fußteilen im Bereich der beiden Kopfenden einer höhenverstellbaren Tischplatte aufweist, die aus Röntgenstrahlen durchlässigem Material besteht, der jedoch nicht mobil ist und durch die beidseitige Anordnung der Hubsäulen die freie Zugänglichkeit des Raumes unter der Tischplatte, etwa für große Bildempfänger eines Röntgensystems, sehr stark behindert.

Ein weiterer Patientenlagerungstisch ist aus EP 0 923 922 A2 bekannt.

Aufgabe der Erfindung ist es, einen fahrbaren Patientenlagerungstisch für die Radiologie, insbesondere für die Röntgendiagnostik zur Verfügung zu stellen, auf dem Patienten während einer Röntgenuntersuchung in Rücken-, Bauch- oder Seitenlage gelagert werden können, wobei der Patientenlagerungstisch die Positionierung und Zentrierung der Patienten im Verhältnis zu einem Bildaufnehmer einer Röntgenvorrichtung ermöglichen soll, in dem der Patientenlagerungstisch vom Anwender einfach und leicht in der Höhe sowie in der Längs- und Querausrichtung der Tischplatte eingerichtet werden kann.

Die Lösung dieser Aufgabe erfolgt in Verbindung der Oberbegriffsmerkmale des Hauptanspruchs erfindungsgemäß mit dessen kennzeichnenden Merkmalen insbesondere dadurch, dass nur eine Hubsäule seitlich unter einem Kopfende einer Tischplatte angeordnet ist, und die Hubsäule auf einem mit Rollen versehenen Fußteil auf einer Linearführung längs verfahrbar gelagert ist und die Tischplatte ihrerseits nur in ihrer Querrichtung schwimmend auf der Hubsäule verstellbar festgelegt ist. Hierdurch wird erreicht, dass der Patientenlagerungstisch schnell und einfach über oder neben einem Bildaufnehmer positioniert werden kann, wobei Aufnahmen in verschiedenen Winkeln durchgeführt werden können und zur Lagerung eines Patienten der Patientenlagerungstisch vom Bildaufnehmer weggezogen werden kann, um etwa eine Höhenverstellung durchzuführen, was das Lagern eines Patienten wesentlich erleichtert.

Dadurch, dass die Linearführung für die Längsbewegung der Tischplatte auf dem Fußteil und nicht an der Tischplatte selbst angeordnet ist, wird es möglich, diese von ansonsten erforderlichen metallischen Führungsbeschlägen vollkommen frei zu halten, so dass nur in Querrichtung eine entsprechende Verstellmechanik unter der Tischplatte angeordnet werden muss, wodurch es gelingt, die Tischplatte rundum aus einem durchstrahlbaren Material zu fertigen, ohne dass im Randbereich oder darunter röntgendichte Bereiche wie von metallischen Längs-Verstellbeschlägen vorhanden sind.

Weitere vorteilhafte Ausgestaltungen der Erfindung ergeben sich mit und in Kombination aus den nachfolgenden Unteransprüchen.

Gemäß einer besonders bevorzugten Ausführungsform der Erfindung weist der Patientenlagerungstisch einen elektrischen Spindelantrieb für die Linearführung sowie einen elektrischen Hubmechanismus für die Hubsäule auf, wobei letzterer in einer Verkleidung der Hubsäule angeordnet ist, ebenso wie zwei Steckplätze für interne elektrische Stromquellen, die vorzugsweise als Akkumulatoren ausgeführt und wechselweise oder gemeinsam angeschlossen sind. Die Ausführung mit zwei Akkus hat zum einen den Vorteil, dass ein vollständig kabelloser Betrieb des Patientenlagerungstisches möglich ist, wobei das Vorhandensein von zwei Steckplätzen die Entnahme eines Akkus zur Wiederaufladung an einem externen Stromnetz ermöglicht, ohne dass die Funktion des Patientenlagerungstisches dadurch nachteilig beeinflusst würde. Auch der Tausch der Akkus kann ohne Ausfallzeiten durchgeführt werden.

Bei einer anderen vorteilhaften Ausführungsform des Gegenstandes der Erfindung ist die Tischplatte über ihre gesamte Länge, insbesondere die frei von der Hubsäule auskragende Länge, aus strahlungsdurchgängigem CFK und vollständig metallfrei ausgebildet, was zu einer hervorragenden Röntgentransparenz der Tischplatte führt und eine optimale Zugänglichkeit für Bildempfänger unter, seitlich, neben oder über dem Patientenlagerungstisch gewährleistet. Vorteilhaft ist des Weiteren die Anordnung eines U-förmigen Bügels um das Kopfende der Tischplatte, der dieses horizontal und beabstandet umläuft und so zum einen als Transporthilfe und Haltegriff, als Rammschutz und vorteilhafterweise auch als Träger der Bedieneinheit dient, da er mit Schaltern und Tastern ausgestattet ist, die zum horizontalen Verschieben der Tischplatte auf der Hubsäule mit einer entsprechenden Verriegelung verbunden sind.

Diese Entriegelung ist vorteilhafterweise elektrisch gesteuert und die Verriegelung der Tischplatte mittels elektromagnetischer Elemente ausgeführt. Zwei Taster sind so in den freien offenen Enden des rohrförmig ausgebildeten Bügels angeordnet, dass sie mechanisch vollkommen geschützt sind, ebenso wie vor einer versehentlichen Bedienung eines Patienten, wodurch eine Zerstörung der Taster oder ungewollte Fehlbedienungen der Verriegelungen ausgeschlossen werden können.

Einer besonders bevorzugten Ausführungsform des Gegenstandes der Erfindung ist mit einem Sensor und einer elektronischen Sicherheitseinrichtung im Bereich der elektrischen Hubmechanik ausgestattet, die während einer Höhenverstellung der Tischplatte eine Belastungsänderung, wie sie etwa bei der Kollision der Tischplatte mit einem im Verstellweg befindlichen Gegenstand erfolgen würde, erkennt und die Tischplatte gegen eine fortgesetzte Bewegung verriegelt oder sogar geringfügig entgegen der letzten Verstellrichtung zurückbewegt. Hierdurch wird die Betriebssicherheit des Patientenlagerungstisches und vor allen Dingen die Zerstörung oder Beschädigung von Röntgenvorrichtungen wirksam und nachhaltig vermieden.

Vorteilhaft ist des Weiteren, dass das Fußteil zwischen den Rollen sehr bodennah verlaufend und insgesamt sehr flach ausgebildet ist, so dass zwischen der Unterseite der Tischplatte und der Oberseite des Fußteils ein größtmöglicher Freiraum aufrechterhalten wird, wodurch auch größere Bildempfänger einer Röntgenvorrichtung problemlos darunter platziert und ausgerichtet werden können.

Nachfolgend ist ein Ausführungsbeispiel der Erfindung anhand von Zeichnungen näher beschrieben. Es zeigen:

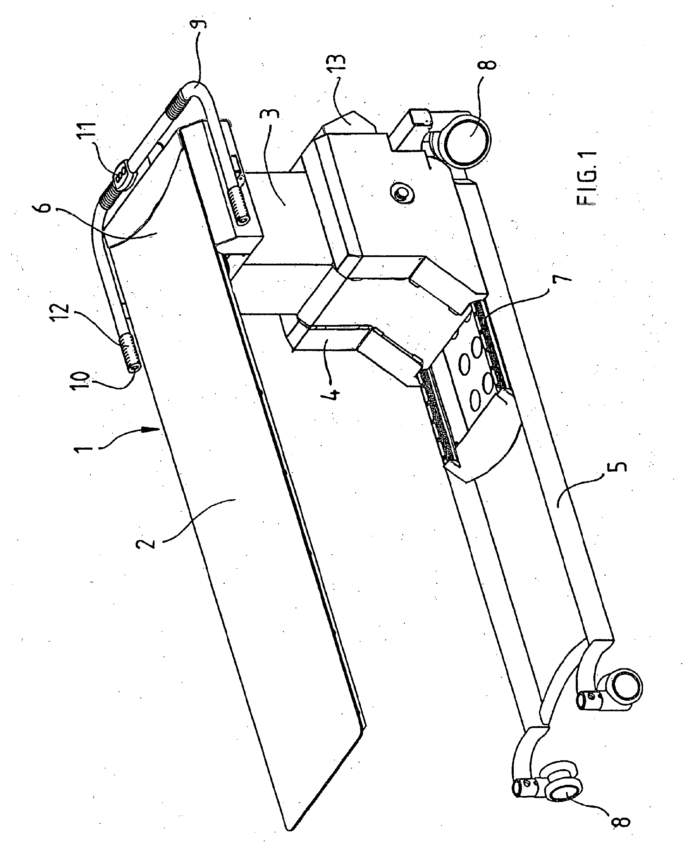

- Fig. 1: eine räumliche Ansicht eines Patientenlagerungstisches,

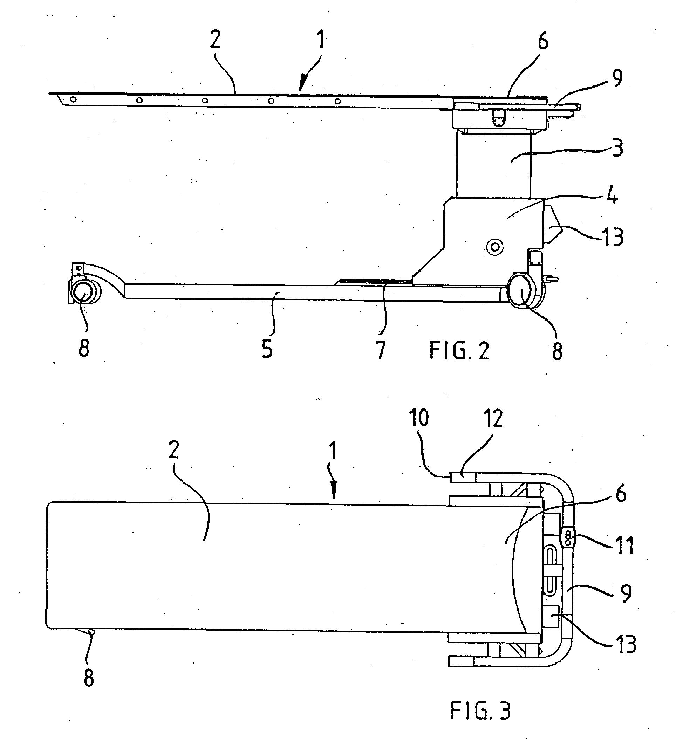

- Fig. 2: eine Seitenansicht eines Patientenlagerungstisches gem. Fig. 1 und
- Fig. 3: eine Draufsicht auf einen Patientenlagerungstisch gem. Fig. 1 und 2.

Der Patientenlagerungstisch 1 besteht aus einer Tischplatte 2, einer unter einem Kopfende der Tischplatte 2 stirnseitig angeordneten Hubsäule 3 mit einer Verkleidung 4 im Bereich des Fußteils 5 des Patientenlagerungstisches 1, welches mit einer Linearführung 7 für die Hubsäule 3 sowie an seinen unterhalb der Kopfenden befindlichen Bereichen mit Rollen 8 ausgestattet ist, wobei das Fußteil 5 rahmenförmig ausgebildet ist und so weit abgesenkt ist, dass es nur kurz oberhalb eines Fußbodens verläuft. In der Verkleidung 4 der Hubsäule 3 sind zwei Akkueinschübe 13 vorgesehen, die den kabellosen Betrieb des Patientenlagerungstisches 1 gewährleisten, wobei für den Betrieb ein Akku ausreichend ist und der zweite extern aufgeladen werden kann. Ein Wechsel eines leeren gegen einen geladenen Akku kann während des Betriebs des Patientenlagerungstisches 1 durch einfaches Umschalten der Stromquellen durchgeführt werden, ohne den Betrieb unterbrechen zu müssen. Das auf der Hubsäule 3 festgelegte Kopfende der Tischplatte 2 wird von einem dieses beabstandet umgebenden U-förmigen Bügel begrenzt, welcher zum einen als Handhabungsgriff bzw. Transporthilfe oder dem Patientenlagerungstisch als Rammschutz dient, zum anderen als Träger der Bedieneinheit, da er mit Schaltern 11 oder Tastern 10 versehen ist, wobei letztere in die offenen Enden der freien Schenkel des als hohlen Rohres ausgebildeten Bügels 9 eingesetzt sind, wodurch diese mechanisch und auch vor ungewollter Betätigung geschützt sind.

Nicht zeichnerisch dargestellt ist die Anordnung eines Sicherheitssensors in Form eines Drucksensors mit der entsprechenden Sicherheitseinrichtung in Form einer Elektronik, die der Erkennung einer plötzlichen Kraftveränderung während der Höhenverstellung der Tischplatte 2 dient, wodurch eine schädigende oder verletzende Kollision der Tischplatte 2 mit anderen feststehenden Bauteilen, wie etwa einer Röntgenvorrichtung, ausgeschlossen werden kann. Der Patientenlagerungstisch 1 kann beispielsweise eine Höhenverstellung von 600 bis 900mm aufweisen und eine Längsbewegung der Hubsäule 3 entlang des Fußteils von 500mm. Die schwimmend gelagerte Tischplatte 2 weist beispielsweise eine Breite von 650mm und eine Länge von 2100mm auf, deren Lagerung einer Querbewegung von +/- 150mm ermöglicht.

Die Tischplatte 2 ist bis auf die Abmessungen der Hubsäule 3 rundum durchstrahlbar und weist keine metallischen Bauteile auf, die das Röntgenbild negativ beeinflussen würden. Die freie Höhe zwischen der Oberseite des Fußteils 5 und der Unterseite der Tischplatte 2 kann beispielsweise 800mm betragen, wodurch die Verwendung auch großer Bildempfänger einer Röntgenvorrichtung möglich wird.

## Patentansprüche

1. Patientenlagerungstisch für die Radiologie, mit einem Fußteil (5), einer auf dem Fußteil (5) angeordneten Hubsäule (3), einer auf der Hubsäule (3) gelagerten Tischplatte (2) aus strahlendurchlässigem Material, wobei die Hubsäule (3) seitlich unter dem Kopfende (6) einer Tischplatte (2) angeordnet und die Tischplatte (2) in ihrer Querrichtung verstellbar auf der Hubsäule (3) gelagert ist, **dadurch gekennzeichnet dass** die Hubsäule auf einer Linearführung (7) längs verfahrbar auf dem mit Rollen (8) versehenen Fußteil (5) gelagert ist.

2. Patientenlagerungstisch nach Anspruch 1, **dadurch gekennzeichnet, dass** die Linearführung (7) einen elektrischen Spindelantrieb und die Hubsäule (3) eine elektrisch angetriebene Hubmechanik und eine Verkleidung (4) aufweist, in der mindestens zwei Steckplätze 13 für interne elektrische Stromquellen angeordnet sind, die als Akkumulatoren ausgebildet und wechselweise oder gemeinsam angeschlossen sind.

3. Patientenlagerungstisch nach einem der vorgenannten Ansprüche,
**dadurch gekennzeichnet, dass** die Tischplatte (2) über ihre gesamte, frei von der Hubsäule (3) auskragenden Länge aus strahlungsdurchlässigem CFK ausgebildet ist.

4. Patientenlagerungstisch nach einem der vorgenannten Ansprüche,
**dadurch gekennzeichnet, dass** um das befestigungsseitige Kopfende (6) der Tischplatte (2) ein dieses horizontal und beabstandet umlaufender U-förmiger Bügel (9) als Transporthilfe, Rammschutz und Bedieneinheit angeordnet ist, der mit Schaltern (11) oder Tastern (10) ausgestattet ist, die zum horizontalen Verschieben der Tischplatte (2) auf der Hubsäule (3) mit einer Entriegelung verbunden sind.

5. Patientenlagerungstisch nach Anspruch 4, **dadurch gekennzeichnet, dass** die Entriegelung elektrisch gesteuert ist und die Verriegelung mittels elektromagnetischer Elemente erfolgt.

6. Patientenlagerungstisch nach Anspruch 4, **dadurch gekennzeichnet, dass** die Taster (10) oder Schalter (11) stirnseitig in den Enden des rohrförmig-Bügels (9) mechanisch oder vor versehentlicher Bedienung geschützt angeordnet sind.

7. Patientenlagerungstisch nach Anspruch 2 **dadurch gekennzeichnet, dass** die elektrische Hubmechanik der Hubsäule (3) mit einem Sensor wie einem Drucksensor und einer elektronischen Sicherheitseinrichtung ausgestattet ist, die eine plötzliche Belastungsänderung der Tischplatte (2) bei deren Höhenverstellung, wie bei einer Kollision mit einem im Verstellweg befindlichen Gegenstand entstehend, detektiert und diese Tischplatte (2) gegen eine fortgesetzte Bewegung verriegelt oder geringfügig entgegen der letzten Verstellrichtung zurückbewegt.

8. Patientenlagerungstisch nach einem der vorgenannten Ansprüche, **dadurch gekennzeichnet, dass** das Fußteil (5) zwischen den Rollen (8) bodennah verlaufend und flach ausgebildet ist und dass zwischen der Unterseite der Tischplatte (2) und der Oberseite des Fußteils (5) ein größtmöglicher Freiraum zur Anwendung großer Bildempfänger erzeugt ist.

## Claims

1. Patient table for radiology, with a base part (5), a lifting column (3) arranged on the base part (5), and a table top (2) mounted on the lifting column (3) and made of radiolucent material, wherein the lifting column (3) is arranged laterally under the head end (6) of a table top (2), and the table top (2) is mounted adjustably in its transverse direction on the lifting column (3), **characterized in that** the lifting column is mounted on a linear guide (7) so as to be longitudinally movable on the base part (5) provided with castors (8).

2. Patient table according to Claim 1, **characterized in that** the linear guide (7) has an electrical spindle drive, and the lifting column (3) has an electrically driven lifting mechanism and a casing (4), in which at least two plug points (13) are arranged for internal electrical power sources which are designed as rechargeable batteries and are connected alternately or together.

3. Patient table according to one of the preceding claims, **characterized in that** the table top (2) is made of radiolucent CFC across its entire length projecting free of the lifting column (3).

4. Patient table according to one of the preceding claims, **characterized in that** the fastening-side head end (6) of the table top (2) is enclosed horizontally and at a distance by a U-shaped bracket (9) serving as transport aid, fender and control unit, which bracket (9) is equipped with switches (11) or buttons (10) which are connected to an unlocking mechanism for the horizontal displacement of the table top (2) on the lifting column (3).

5. Patient table according to Claim 4, **characterized in that** the unlocking mechanism is electrically controlled, and locking takes place by means of electromagnetic elements.

6. Patient table according to Claim 4, **characterized in that** the buttons (10) or switches (11) are arranged at the front in the ends of the tubular bracket (9) for mechanical protection or for protection against inadvertent operation.

7. Patient table according to Claim 2, **characterized in that** the electrical lifting mechanism of the lifting column (3) is equipped with a sensor, such as a pressure sensor, and with an electronic safety device which detects a sudden change of load of the table top (2) during height adjustment thereof, as occurs in the event of a collision with an object located in the path of adjustment, and locks this table top (2) against continued movement or moves it back slightly counter to the last direction of adjustment.

8. Patient table according to one of the preceding claims, **characterized in that** the base part (5) between the castors (8) is close to the floor and flat, and **in that** the largest possible amount of free space, for the use of large image receivers, is created between the underside of the table top (2) and the top of the base part (5).

## Revendications

1. Table de traitement de patient en radiologie, comprenant une partie de base (5), une colonne de levage (3) disposée sur la partie de base (5), un plateau de table (2) supporté sur la colonne de levage (3), en matériau perméable aux rayonnements, la colonne de levage (3) étant disposée latéralement sous l'extrémité de tête (6) d'un plateau de table (2) et le plateau de table (2) étant supporté sur la colonne de levage (3) de manière réglable dans sa direction transversale, **caractérisée en ce que** la colonne de levage est supportée de manière déplaçable longitudinalement sur un guide linéaire (7) sur la partie de base (5) pourvue de roulettes (8).

2. Table de traitement de patient selon la revendication 1, **caractérisée en ce que** le guide linéaire (7) présente un entraînement à broche électrique et la colonne de levage (3) présente un mécanisme de levage à commande électrique et un habillage (4) dans lequel sont disposés au moins deux emplacements de connexion (13) pour des sources internes d'alimentation électrique, lesquelles sont réalisées sous forme d'accumulateurs et sont raccordées en commun ou de manière alternée.

3. Table de traitement de patient selon l'une quelconque des revendications précédentes, **caractérisée en ce que** le plateau de table (2) est réalisé sur toute sa longueur faisant saillie librement depuis la colonne de levage (3) en plastique renforcé par des fibres de carbone perméable aux rayonnements.

4. Table de traitement de patient selon l'une quelconque des revendications précédentes, **caractérisée en ce qu'**autour de l'extrémité de tête (6) du plateau de table (2) côté fixation est disposé un étrier (9) en forme de U entourant celle-ci horizontalement et à distance de celle-ci, réalisé sous forme d'auxiliaire de transport, de protection contre les chocs et d'unité de commande, lequel est muni de commutateurs (11) ou de touches (10) qui sont connectés à un déverrouillage pour le déplacement horizontal du plateau de table (2) sur la colonne de levage (3).

5. Table de traitement de patient selon la revendication 4, **caractérisée en ce que** le déverrouillage est commandé électriquement et le verrouillage s'effectue au moyen d'éléments électromagnétiques.

6. Table de traitement de patient selon la revendication 4, **caractérisée en ce que** les touches (10) ou les commutateurs (11) sont disposés du côté frontal dans les extrémités de l'étrier tubulaire (9) de manière protégée mécaniquement ou contre une commande accidentelle.

7. Table de traitement de patient selon la revendication 2, **caractérisée en ce que** le mécanisme de levage électrique de la colonne de levage (3) est muni d'un capteur tel qu'un capteur de pression et d'un dispositif de sécurité électronique qui détecte une variation de charge soudaine du plateau de table (2) lors de son réglage en hauteur, telle qu'elle se produit dans le cas d'une collision avec un objet se trouvant sur le trajet de déplacement, et qui verrouille ce plateau de table (2) contre la poursuite du mouvement ou le ramène légèrement en arrière dans le sens inverse de ce dernier sens de déplacement.

8. Table de traitement de patient selon l'une quelconque des revendications précédentes, **caractérisée en ce que** la partie de base (5) est réalisée sous forme plate et s'étend à proximité du sol entre les roulettes (8) et **en ce qu'**entre le côté inférieur du plateau de table (2) et le côté supérieur de la partie de base (5) est produit un espace libre le plus grand possible pour l'utilisation de gros récepteurs d'image.
